# EUROPÄISCHE PATENTANMELDUNG

(11) **EP 0 566 110 A2**
(43) Veröffentlichungstag der Anmeldung: **20.10.1993**
(21) Anmeldenummer: 93106116.2
(22) Anmeldetag: 15.04.1993
(51) Int. Cl.: C08L 23/16, C08K 3/00

(54) **Verstärkender Kunstoffstreifen**

(30) Priorität: 17.04.1992 DE 4212877
(71) Anmelder: Karl H. Sengewald GmbH & Co. KG, D-33790 Halle (DE)
(72) Erfinder: Wiedey, Frank, W-4804 Versmold (DE); Neu, Rita, W-4802 Halle/Westf. (DE)
(74) Vertreter: Werner, Hans-Karsten, Dr.Dipl.-Chem.

(57) **Zusammenfassung**

Der verstärkende Kunststoffstreifen zum wiederholbaren Befestigen und Lösen von Klebebändern auf dünnen Kunststoffilmen, insbesondere von flüssigkeitsdichten, außenliegenden Schichten von Wegwerfwindeln, besteht aus einem Polypropylen-Polyethylen-Copolymer mit 3 bis 10 Gew.-%, vorzugsweise 4 bis 7 Gew.-%, Polyethylen, und weist eine Stärke von 30 bis 70 µm, vorzugsweise 45 bis 60 µm, auf, und enthält 5 bis 40 Gew.-%, vorzugsweise 8 bis 15 Gew.-%, Talkum und/oder Kreide, ist jedoch nicht geprägt.

## Beschreibung

Gegenstand der vorliegenden Erfindung ist ein verstärkender Kunststoffstreifen zum wiederholbaren Befestigen und Lösen von Klebebändern auf dünnen Kunststoffilmen, insbesondere von flüssigkeitsdichten, außenliegenden Schichten von Wegwerfwindeln.

Gattungsgemäße verstärkende Kunststoffstreifen sind bekannt, beispielsweise aus der DE-C2-33 38 201. Die dort beschriebenen verstärkenden Kunststoffstreifen bestehen aus Polyester und sind obendrein mit einer Prägung versehen. Diese verstärkenden Kunststoffstreifen haben sich zwar bewährt, jedoch sind sie in der Herstellung sehr aufwendig und teuer, da die geforderte Oberflächenstruktur nur durch eine nachträgliche mechanische Prägung mit einer Prägewalze herstellbar ist.

Die Erfindung hat sich die Aufgabe gestellt, gattungsgemäße verstärkende Kunststoffstreifen zur Verfügung zu stellen, die leichter und preiswerter herstellbar sind und dennoch die guten Eigenschaften aufweisen, die die Kunststoffstreifen gemäß DE-PS 33 38 201 aufweisen.

Diese Aufgabe kann überraschend einfach dadurch gelöst werden, daß dieser Kunststoffstreifen aus einem Polypropylen-Polyethylen-Copolymer mit 3 bis 10 Gew.-%, vorzugsweise 4 bis 7 Gew.-% Polyethylen besteht, eine Stärke von 30 bis 70 µm, vorzugsweise 45 bis 60 µm, aufweist, und 5 bis 40 Gew.-%, vorzugsweise 8 bis 15 Gew.-%, Talkum und/oder Kreide enthält, jedoch nicht geprägt ist.

Vorzugsweise sind diese Kunststoffstreifen verklebbar oder verschweißbar mit elastischen Polymeren oder Copolymeren wie dünnen Filmen aus Polyethylen, Polypropylen, Polyurethan, Ethylenmethacrylat, Ethylenbutylacrylat, Styrol-Butadien-Kautschuk oder Vliesstoffen.

Gegenstand der vorliegenden Erfindung ist somit zunächst der verstärkte Kunststoffstreifen gemäß Ansprüchen 1 und 2. Weiterhin ist Gegenstand der vorliegenden Erfindung die Verwendung von 30 bis 70 µm starken, mit dünnen Polyethylenfilmen verschweißbaren Kunststoffstreifen aus Polypropylen-Polyethylen-Copolymeren mit 3 bis 10 Gew.-%, vorzugsweise 4 bis 7 Gew.-%, Polyethylen und einem Gehalt von 4 bis 40 Gew.-%, vorzugsweisweise 8 bis 15 Gew.-%, Talkum und/oder Kreide als nicht geprägter Verstärkungsstreifen zum wiederholbaren Aufkleben und Lösen von Klebebändern auf den dünnen, flüssigkeitsdichten außenliegenden Außenschichten von Wegwerfwindeln.

Im nachfolgendem Beispiel ist ein verstärkender Kunststoffstreifen näher erläutert, der sich bei der Erprobung sehr gut bewährt hat.

Ein Polypropylen-Polyethylen-Copolymer mit 5 % Polyethylen als Copolymer wird mit 10 Gew.-% Talkum eingehend vermischt und über einen Breitbandextruder zu einem 50 µm starken Film verarbeitet. Der Film wird zu 4 cm breiten Streifen geschnitten. Diese Streifen werden quer zur Windel auf die flüssigkeitsdichte außenliegende Außenschicht aus Polyethylen einer Wegwerfwindel aufgeklebt. Die an den gegenüberliegenden Ecken der Wegwerfwindel befindlichen Klebebänder lassen sich einwandfrei mehrfach befestigen und wieder lösen.

Sofern eine geringere Haftung der Klebebänder gewünscht wird, kann der Gehalt an Talkum und/oder Kreide gesteigert werden. Wird ein weicherer Verstärkungsstreifen gewünscht, kann dieser durch Beimischung von mehr Polyethylen und/oder geringere Dicke hergestellt werden. Demgemäß erhält man aus Copolymeren mit weniger Polyethylen und/oder mit größeren Dicken steifere Verstärkungsstreifen.

## Patentansprüche

1. Verstärkender Kunststoffstreifen zum wiederholbaren Befestigen und Lösen von Klebebändern auf dünnen Kunststoffilmen, insbesondere von flüssigkeitsdichten, außenliegenden Schichten von Wegwerfwindeln, dadurch gekennzeichnet, daß dieser Kunststoffstreifen aus einem Polypropylen-Polyethylen-Copolymer mit 3 bis 10 Gew.-%, vorzugsweise 4 bis 7 Gew.-%, Polyethylen besteht, eine Stärke von 30 bis 70 µm, vorzugsweise 45 bis 60 µm, aufweist, und 5 bis 40 Gew.-%, vorzugsweise 8 bis 15 Gew.-%, Talkum und/oder Kreide enthält, jedoch nicht geprägt ist.

2. Kunststoffstreifen gemäß Anspruch 1, dadurch gekennzeichnet, daß er mit dünnen Polyethylenfilmen klebbar oder verschweißbar ist.

3. Verwendung von 30 bis 70 µm starken, mit dünnen Polyethylenfilmen verschweißbaren Kunststoffstreifen aus Polypropylen-Polyethylen-Copolymeren mit 3 bis 10 Gew.-%, vorzugsweise 4 bis 7 Gew.-%, Polyethylen und einem Gehalt von 4 bis 40 Gew.-%, vorzugsweisweise 8 bis 15 Gew.-%, Talkum und/oder Kreide als nicht geprägter Verstärkungsstreifen zum wiederholbaren Aufkleben und Lösen von Klebebändern auf den dünnen, flüssigkeitsdichten außenliegenden Außenschichten von Wegwerfwindeln.
